# EUROPÄISCHE PATENTANMELDUNG

(11) **EP 1 747 763 A1**
(43) Veröffentlichungstag der Anmeldung: **31.01.2007**
(21) Anmeldenummer: 06013924.3
(22) Anmeldetag: 05.07.2006
(51) Int. Cl.: A61B 18/14

(54) **Injektionsspritze mit einer Injektionsnadel**

(30) Priorität: 29.07.2005 DE 102005035628
(71) Anmelder: Feucht, Peter, 12247 Berlin-Lankwitz (DE)
(72) Erfinder: Feucht, Peter, 12247 Berlin-Lankwitz (DE)
(74) Vertreter: Neubauer, Hans-Jürgen

(57) **Zusammenfassung**

Die Erfindung betrifft eine Injektionsspritze (4) mit einer Injektionsnadel (6) zur Injektion einer Flüssigkeit in ein zu behandelndes Gewebe. Die Injektionsnadel (6) weist eine Metallkanüle (7) auf, die einerseits an einem freien Ende in eine Austrittöffnung (11) mündet und die andererseits mit einem druckbeaufschlagbaren Reservoir (5) für die zu injizierende Flüssigkeit verbunden ist. Erfindungsgemäß weist die Metallkanüle (7) der Injektionsnadel (6) im Verbindungsbereich zum Reservoir (5) einen elektrischen Anschluss (12) für einen elektrochirurgischen Generator in der Art eines HF-Chirurgiegeräts (3) (HochFrequenz-Chirurgiegerät) auf und ist damit als aktive Elektrode bei einer elektrochirurgischen Operation verwendbar. Weiter ist die Metallkanüle (7) in einem Bereich zwischen dem metallisch freien Ende (8) und dem elektrischen Anschluss (12) von einer elektrisch isolierenden Isolierschicht (9) umgeben. Das Ende der Metallkanüle (7) ist als geschlossener Metallkegel (8) ausgebildet, wobei die Metallkanüle (7) eine seitliche, gegenüber der Kanülenspitze zurückversetzte Austrittöffnung (11) aufweist. Mit einer so ausgebildeten Injektionsspritze (4) ist die Injektion einer Flüssigkeit einfach und mit geringem Kraftaufwand auch in hartes Gewebe, insbesondere in einen harten Tumor möglich.

## Beschreibung

Die Erfindung betrifft eine Injektionsspritze mit einer Injektionsnadel für den medizinischen Einsatz zur Injektion einer Flüssigkeit in ein zu behandelndes Gewebe. Die Injektionsnadel weist eine Metallkanüle auf, die einerseits an einem freien Ende in eine Austrittöffnung mündet und die andererseits mit einem druckbeaufschlagbaren Reservoir für die zu injizierende Flüssigkeit verbunden ist.

Bei einer handbetätigbaren allgemein bekannten Injektionsspritze der vorstehenden Art besteht das druckbeaufschlagbare Reservoir aus einer Zylinder-Kolben-Einheit, wobei der Kolben mit einem endseitigen Druckknopf aus dem Zylinder vorsteht und die Metallkanüle gegenüberliegend am Zylinderboden in Axialrichtung angeschlossen ist. Nach dem Einstechen der Injektionsnadel in ein zu behandelndes Gewebe kann im Zylinder enthaltene Flüssigkeit durch Einschieben des Kolbens über die Metallkanüle und deren Austrittöffnung in das Gewebe injiziert werden.

Solche allgemein bekannten Injektionsspritzen werden in einer Vielzahl medizinischer Behandlungen verwendet. Eine Anwendung ist aus der Tumorbekämpfung bekannt, in Verbindung mit einem Hyperthermieverfahrens mit sogenannten magnetischen Flüssigkeiten:

Unter einer magnetischen Flüssigkeit wird hier eine Suspension verstanden, die in der Regel aus Wasser und kleinen magnetsensiblen Teilchen, sogenannten Nano-Teilchen besteht. Solche Nano-Teilchen haben einen Kern aus magnetsensiblen Metallen, insbesondere Eisen oder Eisenoxid von einigen Nanometern Durchmesser, sowie eine Hülle aus einem zuckerähnlichen Stoff.

Beim vorliegenden Verfahren ist es erforderlich, diese Magnetflüssigkeit in den zu behandelnden Tumor einzubringen, wobei dann die Nano-Teilchen wegen ihrer Hülle aus dem zuckerähnlichen Stoff in den Tumorzellen aufgenommen werden. Ein so präparierter Tumor wird dann von außen mittels eines Magnetfeldapplikators mit einem hochfrequenten Wechselfeld befeldet. Energie aus dem hochfrequenten Wechselfeld wird durch Einwirkung auf die Metallkerne der Nano-Teilchen in mechanische Bewegungsenergie und damit in Wärme umgesetzt, wodurch die entsprechenden Tumorzellen selektiv durch die Erwärmung bis etwa 44°C geschwächt oder bei höheren Temperaturen durch Ablation direkt vernichtet werden können. Ein für ein solches Hyperthermieverfahren geeigneter Magnetfeldapplikator ist aus der EP 1 102 609 B1 bekannt.

Bei der Injektion einer magnetischen Flüssigkeit in einen Tumor können Probleme auftreten, wenn der Tumor sehr hart ist. In einem solchen Fall kann die Injektionsnadel gegebenenfalls nicht in den Tumor eindringen und kann bei hoher Kraftanwendung brechen, was erhebliche Beeinträchtigungen eines Patienten nach sich zieht. Zudem besteht die Gefahr, dass bei einer Verletzung des Tumors entartete Tumorzellen in gesundes Gewebe streuen können.

Zudem ist aus der DE 32 25 221 C2 ein elektrochirurgischer Generator zum Anschluss an ein elektrochirurgisches Operationsinstrument bekannt. Ein solcher Generator umfasst eine Gleichstromquelle, eine Ausgangsschaltung, an deren Ausgang Elektroden angeschlossen sind, die hochfrequente Ströme einem zu behandelnden Gewebe zuführen und eine Steuereinrichtung zur Auswahl der gewünschten Stromform und damit der Operationsart. Elektrochirurgische Operationen werden mit einer sogenannten aktiven Elektrode ausgeführt, die einen kleinen Querschnitt aufweist, damit die elektrische Leistung und die chirurgischen Wirkungen auf einen kleinen kontrollierten Bereich begrenzt werden. Der Rückführpfad des Hochfrequenzstroms vom Gewebe zum Generator erfolgt über eine passive Elektrode als sogenannte Patientenplatte, die eine große Fläche aufweist, damit an der Stelle des Stromübergangs vom Körper des Patienten auf diese Elektrode keine elektrochirurgischen Wirkungen auftreten. Das elektrochirurgische Operationselement ist dabei durch die aktive Elektrode gebildet. Es werden im wesentlichen vier chirurgische Operationsarten unterschieden: Dissikation, Fulguration, Schneiden und Schneiden mit Hämostase, wobei die Operationsart Dissikation auch als Koagulation bezeichnet wird. Diesen vier Operationsarten sind unterschiedliche Stromformen mit unterschiedlichen Spannungspegeln mit unterschiedlichen Wellenverläufen und mit unterschiedlichen dem Gewebe zugeführten Energien zugeordnet.

Weiter ist eine Elektrode für elektromedizinische Zwecke, vorzugsweise als Koagulationseleketrode bekannt (DE 651 428 A), zum Anschluss an einen Hochfrequenzapparat, wobei diese Elektrode aus einer metallischen Hohlnadel besteht, die zum Absaugen von eitrigen Flüssigkeiten eingerichtet ist. Diese Hohlnadel kann wahlweise mit einem Spritzenkörper oder über ein Zwischenstück mit dem Hochfrequenzapparat verbunden werden, wobei über das Zwischenstück die Hohlnadel geschlossen wird. Die Hohlnadel wird ohne elektrische Wirkung beispielsweise in einen relativ weichen Abszess eingestochen und daraus eitrige Flüssigkeit abgesaugt. Nach Abnahme eines Spritzenkörpers und Anschluss an den Hochfrequenzapparat wird die Hohlnadel zurückgezogen, wobei die Wandung des Rückzugkanals koaguliert, d. h. verschlossen wird.

Aufgabe der Erfindung ist es, eine bekannte Injektionsspritze mit einer Injektionsnadel so weiterzubilden, dass mit dieser Flüssigkeit, insbesondere eine magnetische Flüssigkeit für ein Hyperthermieverfahren, in relativ hartes Gewebe mit geringer Kraftaufwendung und ohne Gefahr eines Nadelbruchs injizierbar ist.

Diese Aufgabe wird mit den Merkmalen des Anspruchs 1 gelöst.

Gemäß Anspruch 1 weist die Metallkanüle der Injektionsnadel im Verbindungsbereich zum Reservoir einen elektrischen Anschluss für einen an sich bekannten elektrochirurgischen Generator in der Art eines HF-Chirurgiegeräts (Hoch-Frequenz-Chirurgiegerät) auf, wodurch die Metallkanüle als aktive Elektrode bei einer elektrochirurgischen Operation verwendbar ist. Zudem ist die Metallkanüle in einem Bereich zwischen dem metallisch freien Ende und dem elektrischen Anschluss von einer elektrisch isolierenden Isolierschicht umgeben.

Damit ist es vorteilhaft möglich, für einen Einstich der Injektionsnadel in hartes Gewebe, insbesondere einen harten Tumor unter Anwendung von hochfrequentem Wechselstrom aus einem HF-Chirurgiegerät anzuschneiden, wobei jedoch anstelle eines elektrochirurgischen Skalpells die Injektionsnadel selbst verwendet wird. Die Injektionsnadel hat somit hier die Funktion eines elektrochirurgischen Operationsinstruments, mit dem unter Einbringung hochfrequenter elektrischer Energie über die Nadelspitze in das Gewebe das Eindringen der Injektionsnadel leichtgängig ermöglicht wird. In der weiteren Funktion wird nach dem Eindringen der Injektionsnadel in das harte Gewebe der Flüssigkeitskanal zur Injektion der Flüssigkeit durch die Metallkanüle zur Verfügung gestellt.

Durch die elektrisch isolierende Isolierschicht im Anschluss an das metallisch freie Ende wird vorteilhaft erreicht, dass nur dort an der Injektionsnadelspitze die elektrochirurgische Wirkung zur Schaffung eines Nadeleindringkanals im Gewebe besteht. Ohne eine solche Isolierschicht würde die elektrochirurgische Schneidwirkung in unerwünschter Weise auch über die gesamte eingebrachte Injektionsnadellänge auftreten mit einer ungünstigen Beeinträchtigung von gesundem Gewebe.

Für die konkrete Anwendung der erfindungsgemäßen Injektionsspritze ist deren elektrischer Anschluss mit dem Anschluss für eine aktive Elektrode eines HF-Chirurgiegeräts zu verbinden. Die Rückleitung des Hochfrequenzstroms erfolgt über eine großflächige passive Patientenelektrode, die mit dem entsprechenden Anschluss des HF-Chirurgiegeräts zu verbinden ist. Die Injektionsnadel kann bereits von vorneherein mit Hochfrequenzenergie beaufschlagt werden oder ohne eine solche Beaufschlagung vorerst durch weiches Gewebe bis an ein hartes Gewebe, insbesondere einen harten Tumor herangeführt werden, wonach dann für das weitere Eindringen eine Beaufschlagung mit Hochfrequenzenergie erfolgt.

Zudem ist das metallisch freie Ende der Metallkanüle als Metallkegel mit einer geschlossenen Kanülenspitze ausgebildet. Zudem weist die Metallkanüle eine seitliche, gegenüber der Kanülenspitze in Axialrichtung zurückversetzte Austrittöffnung auf. Durch die Kegelspitze ist gewährleistet, dass auch ohne Aufbringung von HF-Energie die Injektionsnadel in normales Gewebe eindringen kann und gegebenenfalls nach dem Durchdringen einer harten Tumorwand mittels HF-Energie im Inneren des Tumors auch ohne HF-Energie weitergeführt werden kann. Durch die geschlossene Kanülenspitze und die nach der Kanülenspitze zurückversetzt angeordnete seitliche Austrittöffnung wird deren unerwünschte Verstopfung durch Abbauprodukte des Schneidvorgangs verhindert. Dazu liegt ein bevorzugter Bereich der Austrittöffnung nach Anspruch 2 im Bereich des vorderen Endes der Isolierschicht unmittelbar nach dem Metallkegel.

Besonders vorteilhaft ist eine solche Injektionsspritze bei einem Hyperthermieverfahren zur Einbringung magnetischer Flüssigkeiten in einen harten Tumor einsetzbar.

Gemäß Anspruch 3 ist anschließend an den Metallkegel der Außendurchmesser der Metallkanüle entsprechend der Isolierschichtdicke dergestalt reduziert, dass vom Metallkegel zur Isolierschicht eine axial stufenfrei durchgehende glatte Außenfläche der Injektionsnadel gebildet ist. Durch eine solche glatte Außenfläche wird das Eindringen in das Gewebe erleichtert.

In einer konstruktiv bevorzugten Ausführungsform nach Anspruch 4 ist die Isolierschicht durch ein Isolierrohr, insbesondere durch ein Kunststoffrohr gebildet, in das die Metallkanüle formschlüssig eingesteckt ist.

Die Isolierschicht kann jedoch auch durch andere an sich bekannte Maßnahmen, wie durch eine dauerhafte Beschichtung hergestellt sein.

Zweckmäßig wird der elektrische Anschluss für das HF-Chirurgiegerät nach Anspruch 5 in der Art eines HF-Steckers ausgeführt, wodurch das HF-Chirurgiegerät einfach und wahlweise lösbar an die erfindungsgemäße Injektionsspritze bzw. die Metallkanüle anschließbar ist. Dabei ist als Berührungsschutz auf eine ausreichende Isolierung der Verbindung zu achten. Zudem sind an sich bekannte Maßnahmen gegen ein unbeabsichtigtes Lösen der Verbindung zu treffen.

Das druckbeaufschlagbare Reservoir für die zu injizierende Flüssigkeit, kann nach Anspruch 6 in an sich bekannter Art als Spritzenkörper durch eine handbetätigbare Zylinder-Kolben-Einheit ausgeführt sein, so dass sich dadurch der übliche allgemein bekannte Aufbau einer Injektionsspritze ergibt. Die Modifikation zur erfindungsgemäßen Injektionsspritze ist dadurch der Injektionsnadel zugeordnet, so dass gegebenenfalls einfach und kostengünstig handelsübliche Spritzenkörper mit einer erfindungsgemäß modifizierten Injektionsnadel kombinierbar sind.

Nach Anspruch 7 ist der Spritzenkörper isoliert ausgeführt, damit der Chirurg oder der Patient bei einer Berührung mit den Spritzenteilen keine Verbrennungen durch hochfrequente Ströme erleiden.

Insbesondere bei mehrfach zu verwendenden Injektionsspritzen ist es für Sterilisationszwecke vorteilhaft, wenn gemäß Anspruch 8 die Kolbenzylindereinheit und/oder die Metallkanüle und/oder die Isolierschicht als Isolierrohr und/oder der elektrische Anschluss lösbar miteinander verbunden sind, so dass diese Teile als Einzelteile sterilisierbar und bei Bedarf wieder zusammensetzbar sind.

Mit den Merkmalen des Anspruchs 9 werden vorteilhafte Einwegausführungen beansprucht.

Anhand einer Zeichnung wird die Erfindung näher erläutert.

Es zeigen:
- Fig. 1: eine an ein HF-Chirurgiegerät angeschlossene Injektionsspritze und einen Patienten in schematischer Darstellung, und
- Fig. 2: einen Längsschnitt durch eine Injektionsnadel der Injektionsspritze nach Fig. 1.

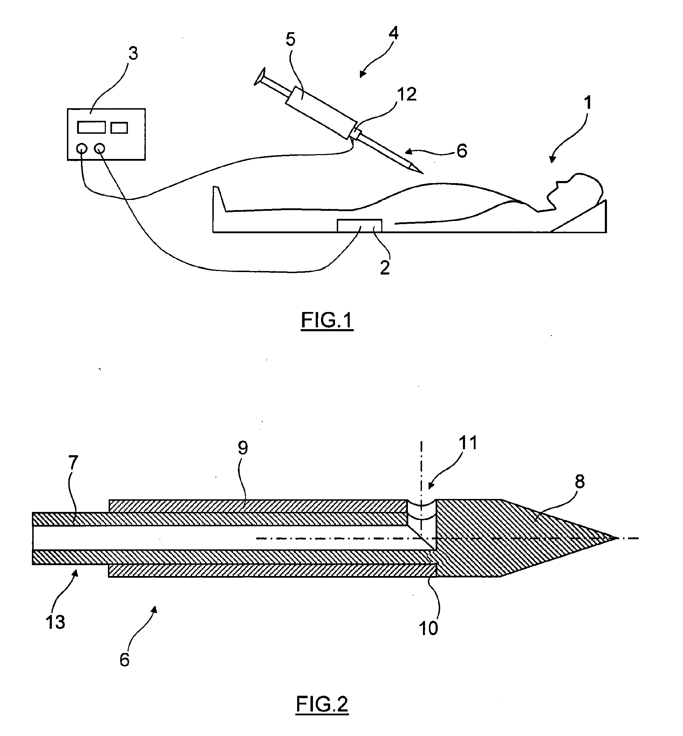

In Fig. 1 ist ein liegender Patient 1 dargestellt, der Kontakt mit einer großflächigen Patientenplatte 2 als passive Elektrode hat, die an einen entsprechenden Anschluss eines HF-Chirurgiegeräts 3 angeschlossen ist. An einen Anschluss des HF-Chirurgiegeräts für eine aktive Elektrode ist eine (gegenüber dem Patienten vergrößert dargestellte) erfindungsgemäße Injektionsspritze 4 angeschlossen.

Die Injektionsspritze 4 besteht aus einer Zylinder-Kolben-Einheit 5 als druckbeaufschlagbares Reservoir für eine zu injizierende Flüssigkeit und aus einer Injektionsnadel 6 deren genauer Aufbau aus dem Längsschnitt nach Fig. 2 ersichtlich ist.

Die Injektionsnadel 6 besteht aus einer Metallkanüle 7, deren freies Ende als Metallkegel 8 mit einer geschlossenen Kanülenspitze ausgebildet ist. Die Metallkanüle 7 ist formschlüssig in ein Isolierrohr 9 eingesteckt dergestalt, dass durch einen Absatz 10 im Außendurchmesser der Metallkanüle 7 nach dem Metallkegel 8 eine durchgehende glatte Außenfläche der Injektionsnadel 6 gebildet ist.

Die Metallkanüle 7 weist eine seitliche gegenüber der Spitze des Metallkegels 8 zurückversetzte Austrittöffnung 11 auf, die im Bereich des vorderen Endes des Isolierrohrs 9 unmittelbar nach dem Metallkegel 8 angeordnet ist.

Die Metallkanüle 7 steht in Richtung auf die Zylinder-Kolben-Einheit 5 für einen dortigen mechanischen und strömungsmäßigen Anschluss sowie zur Anbringung eines HF-Steckeranschlusses 12 mit einem Überstand 13 über die Länge des Isolierrohrs 9 vor.

Die Anordnung hat folgende Funktion: Beim Patienten 1 soll mittels der Injektionsspritze 4 magnetische Flüssigkeit als Suspension aus Wasser und magnetischsensitiven Nano-Teilchen in einen harten Tumor eingebracht werden. Dazu wird die Injektionsnadel 6 zuerst durch das vor dem Tumor liegende normale Gewebe gestochen bis vor eine harte Tumorwand, wobei die Spitze des Metallkegels 8 das Eindringen erleichtert. Nun wird das HF-Chirurgiegerät 3 aktiviert, wodurch die elektrochirurgische Wirkung am Metallkegel 8 für einen kraftreduzierten Einstich durch die harte Tumorwand hervorgerufen wird. Der Metallkegel 8 wirkt dabei als aktive Elektrode mit einer Leistungsbündelung an der kleinflächigen Kegelspitze. Der Rückfluss der Elektroenergie zum HF-Chirurgiegerät wird über die Patientenplatte 2 als passive Elektrode durchgeführt.

Nach dem Einstechen in den Tumor kann die zu injizierende Flüssigkeit durch Betätigung der Zylinder-Kolben-Einheit 5 über die seitliche Austrittöffnung 11 injiziert werden, wobei die seitliche Anordnung der Austrittöffnung 11 eine Verstopfung, insbesondere durch Abbauprodukte des Schneidvorgangs verhindert.

## Patentansprüche

1. Injektionsspritze (4) mit einer Injektionsnadel (6) zur Injektion einer Flüssigkeit in ein zu behandelndes Gewebe, wobei die Injektionsnadel (6) eine Metallkanüle (7) aufweist, die einerseits an einem freien Ende in eine Austrittöffnung (11) mündet und die andererseits mit einem druckbeaufschlagbaren Reservoir (5) für die zu injizierende Flüssigkeit verbunden ist,
**dadurch gekennzeichnet,**
**dass** die Metallkanüle (7) der Injektionsnadel (6) im Verbindungsbereich zum Reservoir (5) einen elektrischen Anschluss (12) für einen elektrochirurgischen Generator in der Art eines HF-Chirurgiegeräts (3) (Hoch-Frequenz-Chirurgiegerät) aufweist und damit als aktive Elektrode bei einer elektrochirurgischen Operation verwendbar ist,
**dass** die Metallkanüle (7) in einem Bereich zwischen dem metallisch freien Ende (8) und dem elektrischen Anschluss (12) von einer elektrisch isolierenden Isolierschicht (9) umgeben ist,
**dass** das metallisch freie Ende der Metallkanüle (7) als Metallkegel (8) mit einer geschlossenen Kanülenspitze ausgebildet ist, und
**dass** die Metallkanüle (7) eine seitliche, gegenüber der Kanülenspitze in Axialrichtung zurückversetzte Austrittöffnung (11) aufweist.

2. Injektionsspritze nach Anspruch 1, **dadurch gekennzeichnet, dass** die Austrittöffnung (11) im Bereich des vorderen Endes der Isolierschicht (9) unmittelbar nach dem Metallkegel (8) angeordnet ist.

3. Injektionsspritze nach Anspruch 1 oder 2, **dadurch gekennzeichnet, dass** anschließend an den Metallkegel (8) der Außendurchmesser der Metallkanüle (7) entsprechend der Isolierschichtdicke dergestalt reduziert (10) ist, dass vom Metallkegel (8) zur Isolierschicht (9) eine axial stufenfrei durchgehende Außenfläche der Injektionsnadel (6) gebildet ist.

4. Injektionsspritze nach einem der Ansprüche 1 bis 3, **dadurch gekennzeichnet, dass** die Isolierschicht durch ein Isolierrohr (9) gebildet ist, in das die Metallkanüle (7) formschlüssig eingesteckt ist.

5. Injektionsspritze nach einem der Ansprüche 1 bis 4, **dadurch gekennzeichnet, dass** der elektrische Anschluss einen HF-Steckeranschluss (12) aufweist.

6. Injektionsspritze nach einem der Ansprüche 1 bis 5, **dadurch gekennzeichnet, dass** das druckbeaufschlagbare Reservoir aus einer Zylinder-Kolben-Einheit (5) als Spritzenkörper besteht, wobei der Kolben für eine Handbetätigung aus einem Zylinder vorsteht und gegenüberliegend am Zylinderboden in Axialrichtung die Metallkanüle (7) angeschlossen ist.

7. Injektionsspritze nach Anspruch 6, **dadurch gekennzeichnet, dass** der Spritzenkörper isoliert ausgeführt ist.

8. Injektionsspritze nach Anspruch 6 oder 7, **dadurch gekennzeichnet, dass** die Kolben-Zylinder-Einheit (5) und/oder die Metallkanüle (7) und/oder die Isolierschicht (9) und/oder der elektrische Anschluss (12) lösbar miteinander verbunden sind.

9. Injektionsspritze nach Anspruch 6 oder 7, **dadurch gekennzeichnet, dass** die Injektionsspritze (4) insgesamt als Einwegartikel oder der Spritzenkörper (5) als Einwegartikel oder eine damit verbindbare Injektionsnadel (6) als Einwegartikel ausgeführt ist, wobei bei der Injektionsnadel (6) das Anschlusskabel für deren Funktion als aktive Elektrode direkt mit der Metallkanüle (7) verbunden ist und diese zusammen mit der Anschlussstelle isoliert ist.
